(19) **Europäisches Patentamt / European Patent Office / Office européen des brevets**

(11) **EP 1 809 237 B1**

(12) **EUROPEAN PATENT SPECIFICATION**

(45) Date of publication and mention of the grant of the patent:
**31.12.2008 Bulletin 2009/01**

(21) Application number: **05795930.6**

(22) Date of filing: **10.10.2005**

(51) Int Cl.:
*A61K 9/00* (2006.01)     *A61K 9/107* (2006.01)

(86) International application number:
**PCT/EP2005/011650**

(87) International publication number:
**WO 2006/050838 (18.05.2006 Gazette 2006/20)**

(54) **OPHTHALMIC OIL-IN-WATER TYPE EMULSION WITH STABLE POSITIVE ZETA POTENTIAL**

OPHTHALMISCHE ÖL-IN-WASSER-EMULSION MIT STABILEM POSITIVEM ZETA-POTENTIAL

ÉMULSION HUILE-DANS-EAU À BASSE CONCENTRATION DE L'AGENT CATIONIQUE ET AU POTENTIEL DE ZÉTA POSITIF

(84) Designated Contracting States:
**AT BE BG CH CY CZ DE DK EE ES FI FR GB GR HU IE IS IT LI LT LU LV MC NL PL PT RO SE SI SK TR**

(30) Priority: **09.11.2004 EP 04292645**
**18.11.2004 US 991346**

(43) Date of publication of application:
**25.07.2007 Bulletin 2007/30**

(73) Proprietor: **Novagali Pharma S.A.**
**91000 Evry (FR)**

(72) Inventors:
• **BAGUE, Séverine**
**F-91310 Linas (FR)**
• **PHILIPS, Betty**
**F-92160 Antony (FR)**
• **RABINOVICH-GUILATT, Laura**
**F-75015 Paris (FR)**
• **LAMBERT, Gregory,**
**Les Murs Blancs**
**F-92290 Chatenay Malabry (FR)**
• **GARRIGUE, Jean-Sébastien**
**Bâtiment 4, F-91370 Verrières Le Buisson (FR)**

(74) Representative: **de Mareüil-Villette, Caroline**
**Cabinet Plasseraud**
**52 rue de la Victoire**
**75440 Paris Cedex 09 (FR)**

(56) References cited:
EP-A- 0 696 452      EP-A- 0 878 197
EP-A- 1 611 879      WO-A- 03/053405
US-A- 6 007 826      US-A1- 2003 108 626

• CHOI W-J ET AL: "Low toxicity of cationic lipid-based emulsion for gene transfer" BIOMATERIALS, ELSEVIER SCIENCE PUBLISHERS BV., BARKING, GB, vol. 25, no. 27, 25 February 2004 (2004-02-25), pages 5893-5903, XP004511128 ISSN: 0142-9612 cited in the application & ANONYMUS: INTERNET ARTICLE, [Online] 25 February 2004 (2004-02-25), page 1, XP002323251 Retrieved from the Internet: URL: doi:10.1016/j.biomaterials.2004.01.031 > [retrieved on 2005-04-05]
• OTT G ET AL: "A cationic sub-micron emulsion (MF59/DOTAP) is an effective delivery system for DNA vaccines" JOURNAL OF CONTROLLED RELEASE, ELSEVIER SCIENCE PUBLISHERS B.V. AMSTERDAM, NL, vol. 79, no. 1-3, 19 February 2002 (2002-02-19), pages 1-5, XP004340908 ISSN: 0168-3659 cited in the application
• OGAWA SATOSHI ET AL: "Production and characterization of O/W emulsions containing cationic droplets stabilized by lecithin-chitosan membranes." JOURNAL OF AGRICULTURAL AND FOOD CHEMISTRY. 23 APR 2003, vol. 51, no. 9, 23 April 2003 (2003-04-23), pages 2806-2812, XP002323017 ISSN: 0021-8561 cited in the application
• TAMILVANAN S ET AL: "The potential of lipid emulsion for ocular delivery of lipophilic drugs" EUROPEAN JOURNAL OF PHARMACEUTICS AND BIOPHARMACEUTICS, ELSEVIER SCIENCE PUBLISHERS B.V., AMSTERDAM, NL, vol. 58, no. 2, 1 June 2004 (2004-06-01), pages 357-368, XP004526318 ISSN: 0939-6411

**EP 1 809 237 B1**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention concerns ophthalmic cationic oil-in water type emulsions having a zeta potential remaining positive overtime.

By "ophthalmic emulsion", it is meant an emulsion which is suitable for an ocular application and which may have a pharmaceutical effect or a cosmetic effect.

Emulsions according to the invention have a zeta potential remaining positive overtime that is, they are stable overtime.

Stability is defined as the extent to which a product retains, within specified limits and throughout its period of storage and use (i.e., its shelf life), the same properties and characteristics that it possessed at the time of manufacture. The purpose of stability testing is to provide evidence on how the quality of a drug substance or drug product varies overtime under the influence of a variety of environmental factors such as temperature, humidity and light, and enables recommended storage conditions, re-test periods and shelf lives to be established.

Although real-time stability studies include an evaluation of those factors that ultimately affect the expiration date of the drugs, they are time and cost-consuming. Conventionally, accelerated stability studies are used for predicting the shelf life of pharmaceutical products. Such accelerated studies subject the systems to a temperature of 40°C during at least 6 months.

In order to understand the intrinsic stability mechanism of the system by establishing degradation pathways and identifying the likely degradation products, and thus to adjust the analytical procedures to be used, the Applicant has developed stress stability testing during which the emulsions are subjected to extreme conditions that is a temperature of 80°C during specified period of time.

Mathematical extrapolations, such as the Arrhenius equation, are then used to calculate the product's predicted shelf life. Application of Arrhenius equation in pharmaceutical stability testing is straightforward. In the isothermal method, the system to be investigated is stored under several high temperatures with all other conditions fixed. Excess thermal exposure accelerates the degradation and thus allows the rate constants to be determined in a shorter time period.

In recent years, oil-in-water type emulsions, in particularly emulsions having droplets of a submicron size (hereinafter "submicron emulsions") gained increasing importance, in particular as vehicles for delivery of hydrophobic drugs.

However, stabilizing emulsions, including submicron emulsions, may be a concern for one skilled in the art. One known approach to stabilize an emulsion is to confer an electrostatic charge to the droplets surface which will result in droplet repulsion and less droplet coalescence. Colloidal particles dispersed in a solution are electrically charged due to their ionic characteristics and dipole attributes. This charge, which can be negative resulting in anionic emulsions or positive producing cationic emulsions (Klang et al., Pharm. Dev. Technology 2000, 5, 521-532) is known in the art as the "zeta potential". The zeta potential is a measure of the magnitude of the repulsion or attraction between particles (Washington, Adv. Drug Deliv. Reviews 1996, 20:131-145).

Formulations of submicron emulsions reported in the literature are usually based on a combination of lecithins which are mixtures of phospholipids of various compositions obtained from natural sources, non-ionic or ionic surfactants and of oil such as vegetable oil. Lecithins generally comprise as major components phosphatidylcholine, which is neutral over a wide pH range, negatively charged phospholipids such as phosphatidylserine and phosphatidic acid and positively charged phospholipids such as phosphatidylethanolamine. As a consequence of their composition, the colloid particles in most available phospholipid-based emulsions are negatively charged. Addition of enough amounts of cationic agents such as stearylamine, oleylamine, chitosan, {N-[i-(2,3-dioleoyloxy)propyl] -N, N,N-trimethyiammonium (DOTAP) or others can reverse this surface charge and produce a positively-charged colloid, as reflected by their zeta potential (Rabinovich-Guilatt et al., Chem Phys Lipids 2004, 131:1-13; Liu et al., Pharm. Res. 1996, 13:1856- 1860, Klang et al., Int. J. Pharm. 1996, 132:33-44).

In all phospholipid-containing colloids (e.g. liposomes or emulsions), a significant decrease in zeta potential is observed overtime, due to the hydrolysis of phospholipids into free fatty acids (Zuidam and Crommelin, J Pharm Sci 1995, 84:1113-1119) which can be a source of toxic side effects following administration (Varveri et al., J. Photochem. Photobiol. A 1995, 91:121-124). In cationic phospholipids colloids, the decrease in zeta potential evidences that the system is not completely chemically stable (Tamilvanan et al., STP Pharma Sciences 2001, 11:421-426) and in some cases could result in the physical destabilization of the formulation as reflected by the droplet size.

For example, in chitosan cationic formulations containing 0.25-1.5% chitosan, 0-1.5% phospholipids, 0-2.5% poloxamer in a castor: soybean oil phase, only the formulation containing poloxamer with chitosan displayed good stability during autoclaving, while the coexistence of chitosan and phospholipids resulted in a destabilization of the emulsion during sterilization. According to the authors, the interaction between the positively charged chitosan with negatively-charged phospholipids which resulted in a damaged emulsifier film around the oil droplets provoked the coalescence of the droplets (Int. J Pharm. 1999, 183:175-84). These emulsions were evaluated further for their antimicrobial activity for mucosal or parenteral administration (Eur. J. Pharm. Biopharm. 2002, 53:115- 23).

Of particular interest are the following patents dealing with cationic emulsions for topical ocular administration:

US Patent 6,007,826 discloses a cationic oil-in-water emulsion which comprises colloid particles with a positively charged interfacial film. The interfacial film is formed by cationic lipids (0.05-3% by weight) such as $C_{10}$-$C_{14}$ primary alkylamines (disclosed are stearylamine or oleylamine), $C_{10}$-$C_{24}$ primary alkanolamine or a cholesterol betainate; phospholipids (0.5-3%) and non-ionic surfactants from the group consisting of poloxamer, tyloxapol, polysorbate, and polyoxyethylene fatty acid esters (0.05-3%). The concentration of the oil is maintained within the 3-20% range. US Patent 6,007,826 emulsions zeta potential are not stable to thermal stress (see Tamilvanan et al., STP Pharma Sciences 2001, 11:421-426 and Example 12 as given hereafter).

US Patent 6,656,460 to Benita and Lambert describes a method of treating a dry eye condition by topical application of a positive submicronic emulsion containing 0.1-0.5% phospholipids, 0.5-2% emulsifying agent such as poloxamer and benzalkonium chloride as a preservative. Since these preparations contain 0.1 to 0.5% by weight of phospholipids, their zeta potential is expected to decrease at 80°C (see Example 12).

WO 03/053405 discloses an emulsion to form a tear film comprising a non-polar phospholipid, a non-polar oil, a non-toxic emulsifying agent and a cationic lipid which imparts a net positive charge to the tear film.

US 6,007,826 discloses an oil-in-water type emulsion useful as a delivery vehicle of pharmaceutically or cosmetically active hydrophobic substances comprising colloid particles having an oily core surrounded by an interfacial film, said interfacial film comprising:

- a cationic lipid (0.05-2%),
- a non-ionic surfactant (0.05-3%),
- a phospholipid as an anionic surfactant (0.5-3%).

EP 0 696 452 discloses an oil-in-water type nanoemulsion useful as an ophthalmic vehicle, comprising:

- an oil (0.1-10%),
- a poloxamer as a non-ionic surface agent (0.1-10%),
- BAK as a preservative (equal or less than 0.01%),
- optionally an isotonizing agent, a viscosity modifying agent, a stabilizer, a buffer and/or an antioxidant.

EP 0 878 197 discloses a composition comprising difluprednate, oil, water and an emulsifier, for example a surfactant, that can be administered to the eye, nose, ear and the like. The composition may also comprise a preservative such as benzalkonium chloride.

EP 1 611 879 discloses an oil-in-water type emulsion for intra- and peri-ocular injection, comprising a phospholipid, such as lipoïd E80.

Although some of the prior art emulsions may show a good physical stability regarding droplet size, there is still a need for cationic ophthalmic emulsions which are physically stable, and which do not contain a sufficient amount of any substances susceptible of affecting the zeta potential overtime, while presenting a good tolerability for ocular administration.

Substances susceptible of affecting the zeta potential may be phospholipids, and any substances which become negatively charged upon storage.

The amount of substances affecting the zeta potential overtime must be such that at any time, the amount of positive charges in the emulsion is above the amount of negative charges.

By overtime in the meaning of this invention, it is meant a duration exceeding 1 year, preferably exceeding 2 years, more preferably exceeding 3 years. In this invention, if an emulsion meets any of tests A to D requirements, it is believed that said emulsion has a potential zeta that is not affected overtime.

By "good tolerability" in the present the invention, it is understood that the ratio therapeutic benefit to ocular discomfort is acceptable by the patient, and preferably similar to a placebo or NaCl 0.9% solution.

It is generally accepted that in order to show good ocular tolerability the cation content within the formulation should not exceed 0.1%, preferably not exceed 0.05% and even more preferably should not exceed 0.03%. Primary amines such as stearylamine or oleylamine were shown to be safe for ocular administration at 0.1% or 0.3% w/v (Klang et al., J. Pharm. Pharmacol. 1994, 46:986-993)

Quaternary amines such as benzalkonium chloride, benzododecinium bromide and benzethonium chloride are allowed by health authorities for ophthalmic administration up to concentration of approximately 0.03% (Furrer et al., Eur. J. Pharm. Biopharm. 2002, 53:263-280). Even though the presence of an important amount of cationic agent in emulsions may succeed in maintaining a stable positive zeta potential over time by cancelling emerging negative entities (see Example 13), such emulsions are not compatible with common recommendation for ophthalmic compositions. Attempts to reduce the cation concentration would lead to destabilization of the emulsions (see Example 14).

It is an object of this invention to propose submicron ophthalmic emulsions including minimal amounts of cationic agent and still having and keeping a stable positive zeta potential overtime.

The present invention relates to an ophthalmic oil-in-water type emulsion, which comprises colloid particles having an oily core surrounded by an interfacial film, said emulsion comprising:

- 0.001 % to 0.1% by weight of at least one cationic agent selected from the group consisting of $C_{10}$-$C_{24}$ primary alkylamines, tertiary- aliphatic amines, quaternary ammonium compounds, cationic lipids, amino alcohols, biguanide salts, cationic polymers and a mixture of two or more thereof,
- less than 1% by weight of at least one non ionic surfactant selected from the group consisting of tyloxapol, polysorbates, polyoxyethylene castor oil derivatives, sorbitan esters, polyoxyl stearates and a mixture of two or more thereof,
- said emulsion having a positive zeta potential and meeting the zeta potential stability Test A requirement as defined hereafter,
- provided that the emulsion does not contain phospholipids.

The emulsions according to the invention are physically stable overtime as defined hereabove and keep a positive zeta potential in the specific measurement conditions as described in Tests A, B, C and/or D.
According to the invention, the emulsions do not contain a sufficient amount of any substances susceptible of affecting the zeta potential overtime.

Zeta potential

Zeta potential measures a physical property which is exhibited by any particle in suspension. Zeta potential can be used to predict behaviour of the suspension in different environments, to optimize the formulations of suspensions and emulsions as well as to predict overtime stability.

In order to avoid the emulsion droplets to merge one with the other and form aggregates of successively increasing size, it is necessary to confer repulsive forces to the particles. One of the means to confer repulsive forces to a colloidal system is by electrostatic or charge stabilization. Electrostatic or charge stabilization has the benefits of stabilizing a system by simply altering the concentration of ions in the system. This is a reversible and inexpensive process.

There might by many origins of this surface charge depending upon the nature of the particle and its surrounding medium but the most important mechanisms are the ionisation of surface groups or the adsorption of charged ions.

The interaction of particles in polar liquids is not governed by the electrical potential at the surface of the particle, but by the effective potential of the particle and its associated ions. To utilize electrostatic control of dispersions, it is the zeta potential of the particle that must be measured rather than its surface charge. Charged particles will attract ions of opposite charge in the dispersant. Ions close to the surface are strongly bound; those further away form a more diffuse region. Within this region is a notional boundary, known as the slipping plane, within which the particle and ions act as a single entity. The potential at the slipping plane is known as the zeta potential. It has long been recognised that the zeta potential is a very good index of the magnitude of the interaction between colloidal particles and measurements of zeta potential are commonly used to assess the stability of colloidal systems. The zeta potential measured in a particular system is dependent on the chemistry of the surface, and also of the way it interacts with its surrounding environment. Therefore zeta potential must always be studied in a well defined environment (specifically pH and ionic strength).

Electrophoretic mobility

An important consequence of the existence of electrical charges on the surface of particles is that they interact with an applied electric field. These effects are collectively defined as electrokinetic effects. If the motion is induced in a particle suspended in a liquid under the influence of an applied electric field, it is more specifically named electrophoresis. When an electric field is applied across an electrolyte, charged particles suspended in the electrolyte are attracted towards the electrode of opposite charge. Viscous forces acting on the particles tend to oppose this movement. When equilibrium is reached between these two opposing forces, the particles move with constant velocity. The velocity is dependent on the strength of electric field or voltage gradient, the dielectric constant of the medium, the viscosity of the medium and the zeta potential. The velocity of a particle in a unit electric field is referred to as its electrophoretic mobility. Zeta potential is related to the electrophoretic mobility by the Henry equation:

$$U_E = \frac{2 \ \varepsilon \ z \ f(\kappa a)}{3\eta}$$

where $U_E$ = electrophoretic mobility, z= zeta potential, $\varepsilon$ = dielectric constant, $\eta$ = viscosity and $f(\kappa a)$=Henry's function.

Electrophoretic determinations of zeta potential are most commonly made in aqueous media and moderate electrolyte concentration. $f(\kappa a)$ in this case is 1.5, and this is referred to as the Smoluchowski approximation. Therefore calculation of zeta potential from the mobility is straightforward for systems that fit the Smoluchowski model, i.e. particles larger than about $0.2\mu m$ (microns) dispersed in electrolytes containing more that 10-3 molar salt. For small particles in low dielectric constant media (eg non-aqueous media), $f(\kappa a)$ becomes 1.0 and allows an equally simple calculation. This is referred to as the Huckel approximation.

Tests A, B, C and D

Test A consists in measuring the stability of the emulsion zeta potential under thermal stress conditions.

Zeta potential of the emulsion is measured at T=0, i.e. as soon as the emulsion has been prepared, the obtained value being named $Z_0$. Glass vials (Type I) of 10ml effective capacity containing between 5-10ml of emulsion and sealed under nitrogen atmosphere (without bubbling) are stored at 80°C.

Then at T=15 hours the zeta potential $Z_{15h}$ is measured.

The value $\delta A = Z_{15h}-Z_0$ is then calculated.

For each measurement of the zeta potential, it is operated as follows:

The zeta potential of the emulsion droplet surface is determined by electrophoretic mobility in an apparatus such as a Malvern Zetasizer 2000 (Malvern Instruments, UK) equipped with suitable software and calibrated with the supplied standard.

The emulsion is diluted in double distilled water if needed in order to obtain the scattering intensity allowing optimal particle detection. The sample count rate should be between 100 to 1000 Pa.s (KCps), in homodyne detection (if heterodyne detection is used, the contribution of the reference beam should be deduced). Three consecutive measurements are performed at 25°C using a constant cell drive of 150mV. The electrophoretic mobility is converted into zeta potential values through the Smoluchowsky equation, using the dielectric constants and viscosity of water. The measured value corresponds to the average of the 3 obtained values.

It is considered that the emulsion meets zeta potential stability Test A if $\delta A$ is less than the standard error of measurements, preferably less than 10mV, and even more preferably less than 5mV.

According to an advantageous embodiment, the ophthalmic emulsion according to the invention meets zeta potential stability Test B.

Test B is similar to Test A except that the emulsion is stored during 48 hours at 80°C, the zeta potential $Z_2$ is measured on after 48 hours and $\delta B = Z_2 -Z_0$ is calculated. The emulsion is considered as meeting the requirements of zeta potential stability test B if $\delta B$ is less than the standard error of measurements, preferably less than 10mV, and even more preferably less than 5mV.

According to a more advantageous embodiment of the invention, the ophthalmic emulsion according to the invention meets zeta potential stability Test C.

Test C is similar to Test A except that the emulsion is stored during 7 days at 80°C, the zeta potential $Z_7$ is measured on day 7 and $\delta C = Z_7 -Z_0$ is calculated. The emulsion is considered as meeting the requirements of zeta potential stability test C if $\delta C$ is less than the standard error of measurements, preferably less than 10mV, and even more preferably less than 5mV.

According to a still more advantageous embodiment of the invention, the ophthalmic emulsion according to the invention meets zeta potential stability Test D.

Test D is similar to Test A except that the emulsion is stored during 14 days at 80°C, the zeta potential $Z_{14}$ is measured on day 14 and $\delta D = z_{14} -z_0$ is calculated. The emulsion is considered as meeting the requirements of zeta potential stability test D if $\delta D$ is less than the standard error of measurements, preferably less than 10mV, and even more preferably less than 5mV.

The concentration of the cationic agent is comprised between 0.001 and 0.1%, preferably between 0.002 and 0.05%, and still more preferably between 0.003 and 0.03% by weight of the total weight of the emulsion (w/w).

Advantageously, the concentration of the oil is not higher than 7%, preferably about 0.5 to 5%, and still more preferably about 1 to 3% by weight of the total weight of the emulsion (w/w).

In another embodiment of the invention, the weight ratio cationic agent/oil is comprised between 0.0025 and 0.06, preferably between 0.005 and 0.04, preferably from 0.01 to 0.02.

In the emulsion according to the invention, the concentration of non-ionic agent is less than 1%, preferably comprised between 0.01 to 0.6% by weight of the total weight of the emulsion (w/w).

In the ophthalmic oil-in-water emulsion according to the invention, the cationic agent is selected in the group consisting of $C_{10}$-$C_{24}$ primary alkylamines, tertiary aliphatic amines, quaternary ammonium compounds, cationic lipids, amino alcohols, biguanide salts, cationic polymers and the mixture of two or more thereof.

The primary amine is preferably selected from the group consisting of oleylamine and stearylamine; the tertiary aliphatic salt can be dimethyl lauramine or diethanolamine, the amino alcohol can be tris (hydroxymethyl) aminomethane.

In a preferred embodiment, the cationic agent is a quaternary ammonium compound preferably selected from the group consisting of benzalkonium halide, lauralkonium halide, cetrimide, hexadecyltrimethylammonium halide, tetradecyltrimethylammonium halide, dodecyltrimethylammonium halide, cetrimonium halide, benzethonium halide, behenalkonium halide, cetalkonium halide, cetethyldimonium halide, cetylpyridinium halide, benzododecinium halide, chlorallyl methenamine halide, myristalkonium halide, stearalkonium halide or a mixture of two or more thereof, halide being preferably chloride or bromide. Advantageously, said cationic agent can be selected from the group comprising benzalkonium chloride, lauralkonium chloride, benzododecinium bromide, benzethenium chloride, hexadecyltrimethylammonium bromide, tetradecyltrimethylammonium bromide, dodecyltrimethylammonium bromide or a mixture of two or more thereof.

The cationic polymer can be chitosan, the cationic lipid can be 1,2-dioleyl-3- trimethylammonium-propane, 1,2-dioleoyl-sn-glycero- phosphatidylethanolamine, cationic glycosphingo-lipids or cationic cholesterol derivatives.

Examples of biguanide salts may be selected from the group comprising chlorhexidine and salts thereof, polyaminopropyl biguanide, phenformin, alkylbiguanide or a mixture of two or more therof.

Examples of non-ionic surfactants which may be included in the emulsion of the invention are tyloxapol, poloxamer such as Pluronic F68LF™ or Lutrol F68, Pluronic L-G2LF™ and Pluronic L62D™ (BASF Wyandotte Corp., Parsippany, N.J., USA), polysorbates such as polysorbate 20 and polysorbate 80, polyoxyethylene castor oil derivatives, sorbitan esters, polyoxyl stearates and a mixture of two or more thereof.

Advantageously, the oil-in-water emulsion according to the instant invention comprises benzalkonium chloride as cationic agent and tyloxapol as one of the non-ionic surfactants.

According to another advantageous mode, the emulsion comprises benzalkonium chloride as cationic agent and tyloxapol and poloxamer as non-ionic surfactants.

According to the invention, the colloidal particles have an average particle size of equal or less than 1 $\mu$m, advantageously equal or less than 300 nm, more advantageously in the range of 100 to 250 nm.

The oil phase of the emulsion may comprise one or more components selected from the group consisting of vegetable oils (i.e. soybean oil, olive oil, sesame oil, cotton seed oil, castor oil, sweet almond oil), mineral oil (i.e. petrolatum and liquid paraffin), medium chain triglycerides (MCT) (i.e. a triglyceride oil in which the carbohydrate chain has about 8-12 carbon atoms), oily fatty acid, isopropyl myristate, oily fatty alcohols, esters of sorbitol and fatty acids, oily sucrose esters, and in general any oily substance which is physiologically tolerated.

The major component of the oily phase will preferably be either vegetable oil and/or MCT. Fatty acids or fatty alcohols may be included in cases where the hydrophobic substance to be carried by the emulsion is not sufficiently soluble in the oily phase.

Examples of MCT oil which may be used in emulsions of the present invention are TCM™ (Société des Oléagineux, France), Miglyol 812™ (Dynamit Novel, Sweden).

Other optional compounds which may be present in the emulsion according to the invention are for example anionic surfactants and various additives such as osmotic pressure regulators, e.g. sucrose, glycerine or mannitol; antioxidants, e.g. alpha-tocopherol, sodium bisulfite, sodium metasulfite, sodium thiosulfate anhydrous, citric acid monohydrate, ascorbyl palmitate and ascorbic acid; or preservatives, e.g. thiomersal, chlorobutanol, benzyl alcohol, phenoxyethanol, phenylethyl alcohol, sorbic acid, EDTA and methyl-, ethyl-, or butyl paraben; said optional compounds may only be added in specific concentrations that do not impair the zeta potential stability.

A preferred pH in the aqueous phase of the emulsion of the invention is 4.0-8.5, 6.0-8.0 being particularly preferred.

This invention also relates to a process for the preparation of an ophthalmic oil-in-water type emulsion according to the invention, which comprises colloid particles having an oily core surrounded by an interfacial film, said emulsion comprising at least one cationic agent, at least one non ionic surfactant said emulsion having a positive zeta potential and meeting zeta potential stability Test A requirements, said process comprising the steps of shear mixing and then high pressure homogenization of the coarse emulsions obtained through mixing of the aqueous and the oily phases.

Ophthalmic emulsions in accordance with the present invention may be formulated into pharmaceutical compositions with various hydrophobic active ingredients for a large number of pharmaceutical applications. Also hydrophilic agents can be administered with these emulsions.

According to the invention, the emulsion may be formulated for ocular administration of said active ingredients. In this oil-in-water emulsion, the water-insoluble drug is solubilized in the internal oil phase, thereby remaining in the preferred molecular state. In addition, the blurred vision caused by oils is minimised by the water in the external phase. Furthermore, the concentration of the drug in the oil phase can be adjusted to maximise thermodynamic activity, thus enhancing drug penetration to deeper tissues.

Consequently, the instant invention provides the use of an oil-in-water emulsion according to the instant invention for the preparation of a medicament useful for preventing or treating ophthalmic disorders.

The invention also concerns ophthalmic formulations comprising an oil-in-water emulsion according to the instant invention and a pharmaceutically acceptable carrier selected from the group comprising eye drop composition, eye ointment, ophthalmic gel.

Said formulations may also comprise a pharmaceutically effective amount of an active ingredient in or within the pharmaceutically acceptable carrier.

The instant invention also provides a method of treatment of ocular conditions comprising a pharmaceutical composition comprising an oil-in-water type emulsion as defined above.

The invention also relates to the use of an oil-in-water emulsion according to the instant invention or of an ophthalmic composition as defined above for the preparation of a medicament for the treatment of ocular conditions.

A wide variety of ocular conditions such as glaucoma, ocular inflammatory conditions such as keratitis, uveitis, intra-ocular inflammation, allergy and dry-eye syndrome ocular infections, ocular allergies, ocular infections, cancerous growth, neo vessel growth originating from the cornea, retinal oedema, macular oedema, diabetic retinopathy, retinopathy of prematurity, degenerative diseases of the retina (macular degeneration, retinal dystrophies), retinal diseases associated with glial proliferation may be prevented or treated using the cationic emulsions according to the present invention.

Some substances suitable for delivery to the eye may include, for example, antibiotics (such as tetracycline, chlortetracycline, bacitracin, neomycin, polymyxin, gramicidin, cephalexin, oxytetracycline, chloramphenicol, kanamycin, rifampicin, tobramycin, gentamycin, ciprofloxacin, aminosides, erythromycin and penicillin, quinolone, ceftazidime, vancomycine imipeneme); antifungals such as amphotericin B and miconazole; antibacterials such as sulfonamides, sulfadiazine, sulfacetamide, sulfamethizole and sulfisoxazole, nitrofurazone and sodium propionate; antivirals, such as idoxuridine, trifluorotymidine, acyclovir, ganciclovir, cidofovir and interferon; antibacterial agents such as nitrofurazone and sodium propionate; non-antibiotic, anti-infection, anti-bacterial or anti-microbial drugs such as iodine based preparation triclosan, chlorhexidine; antiallergenics such as sodium cromoglycate, antazoline, methapyriline, chlorpheniramine, cetirizine, pyrilamine and prophenpyridamine; antiproliferative agents such as thalidomide; synthetic gluocorticoids and mineralocerticoids and more generally hormones forms derivating from the cholesterol metabolism (progesterone, estrogens, androgenic hormones such as testosterone, DHEA and their derivatives); anti-inflammatories such as hydrocortisone, hydrocortisone acetate, dexamethasone, dexamethasone 21-phosphate, fluorocinolene, medrysone, prednisolone acetate, luoromethalone, triamcinolone and triamcinolene acetonide and their derivatives; non-steroidal anti-inflammatories such as salicylate, indomethacin, ibuprofen, diclofenac, flurbiprofen and piroxicam and COX2 inhibitors such as rofecoxib, diclofenac, nimesulide, nepafenac; antineoplastics such as carmustine, cisplatin, mitomycin and fluorouracil; immunological drugs such as vaccines and immune stimulants; insulin, calcitonin, parathyroid hormone and peptide and vasepressin hypothalamus releasing factor; beta adrenergic blockers such as timolol maleate, levobunolol HCl and betaxolol HCl, timolol-base, betaxolol, atenolol, epinephrine, dipivalyl, oxonolol, acetazolamide-base and methazolamide; cytokines, interleukins, and growth factors (growth factors such as epidermal growth factor, fibroblast growth factor, platelet derived growth factor, transforming growth factor beta, ciliary neurotrophic growth factor, glial derived neurotrophic factor, NGF, EPO, P1GF); antibodies or antibodies fragments, oligoaptamers, aptamers and gene fragments (oligonucleotides, plasmids, ribozymes, small interference RNA, nucleic acid fragments, peptides, antisense sequences); immunosuppresants such as cyclosporine, sirolimus and tacrolimus, immunomodulators such as endoxan, , tamoxifene; antithrombolytic and vasodilator agents such as rtPA, urokinase, plasmin, nitric exide denors; antioxidants such as lutein, vitamins and/or their derivatives; and/or optically acceptable salts thereof.

According to an advantageous embodiment, the active substance is at least one immunosuppressive agent, preferably chosen in the group consisting of cyclosporine, preferably cyclosporin A, tacrolimus and sirolimus. Advantageously, in these emulsions the immunosuppressive agents is in an amount of 0.01 to 0.4 %, preferably 0.05 to 0.2% (w/w). Advantageously, these emulsions do not contain phospholipids. Advantageously, these emulsions of the invention contain cyclosporine, sirolimus and/or tacrolimus in a vehicle comprising or consisting of MCT. Without being linked by a theory, the use of MCT, a vegetal oil selected among all, may provide stability and bioavailability to the ophthalmic emulsions of the invention containing at least one immunosuppressive agent, preferably cyclosporine A. MCT has been found to have good solubilizing properties of cyclosporine, which may play a role in the observed improved bioavailability of cyclosporine in the emulsions of the invention. Advantageously, the amount of MCT is from 0.5 to 4 % w/w of the emulsion. Advantageously, the emulsion of the invention contains an immunosuppressive agent, preferably chosen in the group consisting of cyclosporine, preferably cyclosporin A, tacrolimus and sirolimus and MCT and tyloxapol. Advantageously, the amount of tyloxapol is from 0.05 to 0.5 % w/w of the emulsion. Preferably, the weight ratio of immunosuppressive agent to oil is from 0.0125 to 0.1. In a particular embodiment of the emulsion the weight ratio of immunosuppressive agent to oil is from 0.083 to 0.1. In another particular embodiment of the emulsion, the weight ratio of immunosuppressive agent to oil is from 0.0125 to 0.05. The emulsions of the invention containing at least one immunosuppressive agent are particularly useful to treat dry eye conditions, in particular keratoconjunctivitis sicca (KCS), atopic keratoconunctivitis sicca (AKC) and vernal keratoconjunctivitis (VKC).

The invention also relates to the use of an oil-in-water emulsion containing or not an active substance for the preparation of an ophthalmic composition for treating dry-eye conditions.

The emulsion according to the invention may also be included in a delivery device selected from the group comprising lenses, ocular patch, implant or insert.

The drug or active substance may be present in an amount of about 0.0001 to 5% by weight of the emulsion. Depending upon whether the drug is hydrophilic or hydrophobic, it will be physically present in the oily phase or in the aqueous component.

The best mode of making and using the present invention are described in the following examples.

### EXAMPLES

In the following examples, the following abbreviations are used:

CAB: mixture of hexadecytrimethyl ammonium bromide, tetradecyltrimethylammonium bromide and dodecyltrimethylammonium bromide
MCT: TCM™ (Société des Oléagineux, France)
BAK: benzalkonium chloride
BEC: benzethonium chloride
BCB: benzyldimethyldodecylammonium bromide
OA: Oleylamine (Sigma (USA)
SA : Stearylamine (Sigma, USA)
CsA: Cyclosporin A
Cremophor: Cremophor EL (BASF, France)
Lutrol: Lutrol F68 (BASF, France)
Oxypol (Gattefosse, France)
Montane 20 (SEPPIC, France)
Oxypol: Gattefosse (St Priest, France)
Montane 20 (SEPPIC, France)

**Reference example 1: Preparation of cationic emulsions where the cationic agent is CTAB**

Methods:

| Component | Z01EM042 | Z01EM043 |
|---|---|---|
| CTAB (cationic agent) | 0.05% | 0.1% |
| MCT (oil) | 2% | 2% |
| Alpha-tocopherol (antioxidant) | 0.01% | 0.01% |
| Lipoid E80™ (anionic surfactant) | 0.32% | 0.32% |
| Lutrol F68™ (non ionic surfactant) | 0.5% | 0.5% |
| Glycerin (tonicity agent) | 2.25% | 2.25% |
| Water | 94.87% | 94.82% |

The oily phase components were successively weighed in the same beaker and then magnetically stirred under a slight heating (40°C) until a yellow, limpid and slightly viscous phase is obtained. Aqueous phase components were successively weighed in the same beaker and then magnetically stirred under a slight heating (40°C) until a transparent, limpid and fluid phase is obtained. Both phases were heated to 65°C. The coarse emulsion was formed by rapid addition of the aqueous phase in the oily phase and was then rapidly heated to 75°C. The aqueous phase and coarse emulsion beakers were protected by a film to avoid any water evaporation. The emulsion was white and slightly transparent. The emulsion droplet size was then decreased by a 5 minutes high shear mixing with a POLYTRON PT 6100. The emulsion became milky. The emulsion temperature was cooled down to 20°C using an ice bath.

The final emulsion was obtained by homogenization in a microfluidizer (C5, Avestin) using continuous cycles for 5 min at a pressure of 10,000 psi. The emulsion was milky, very fluid and did not adhere on the glass. The emulsion temperature was decreased to 25°C. Its pH was measured and then adjusted to 8.00 using a 0.1 M HCl or 0.1 M NaOH solution. Emulsion was conditioned in tinted glass vials with nitrogen bubbling and then sterilized in an autoclave 20 minutes at 121°C.

**EP 1 809 237 B1**

The mean particle size of the emulsions droplets was determined by quasi-elastic light scattering after dilution in water using a High Performance Particle Sizer (Malvern Instruments, UK). The electrophoretic mobility was measured at 25°C in a Malvern Zetasizer 2000 (Malvern Instruments, UK) following a 1:200 dilution in double distilled water as detailed above.

Results:

|  | Z01EM042 | Z01EM043 |
|---|---|---|
| Droplet size (nm) | 126 | 128 |
| Zeta potential (mV) | 36.4 | 49.5 |

**Example 2**: **Preparation of a cationic emulsion wherein the cationic agent is benzalkonium chloride**

Methods:

| Component | Z01EM042 |
|---|---|
| BAK (cationic agent) | 0.02% |
| MCT (oil) | 1% |
| Alpha-tocopherol (antioxidant) | 0.005% |
| Tyloxapol (non ionic surfactant) | 0.16% |
| Lutrol F68 (non ionic surfactant) | 0.5% |
| Glycerin (tonicity agent) | 2.25% |
| Water | 96.07% |

Preparation according to the process described in Reference Example 1.

Results:

|  | Z01EM093 |
|---|---|
| Zeta potential (mV) | 20.4 |

**Example 3**: **Stability of a cationic emulsion described in example 3**

Methods:

The stability of the autoclaved emulsion (zeta potential) at 80°C was monitored for 15 days.

Results:

| | Z01EM093 | | | | | | |
|---|---|---|---|---|---|---|---|
| T(days) | 0 | 2 | 5 | 7 | 9 | 12 | 14 |
| Zeta potential (mV) | 20.4 | 23.2 | 21.5 | 21.6 | 22.7 | 21.0 | 21.3 |

The zeta potential of the emulsion presented in this example was more stable than previously known formulations (data not shown). Z01EM093 meets zeta potential stability test D requirements.

**Example 4**: **Cationic emulsions wherein the cationic agent is oleylamine**

Methods:

| % w/w | Z01EM165 |
|---|---|
| Oleylamine | 0.05 |
| Mineral oil | 1 |
| LutrolF68 | 0.1 |
| Tyloxapol | 0.3 |
| glycerin | 2.25 |
| Water | Up to 100 |

Preparation according to the process described in Reference Example 1.

Results:

| T(days) | Z01EM165 | |
|---|---|---|
| | Droplet size(nm) | Zeta potential(mV) |
| 0 | 186 | 51.3 |
| 3 | 184 | 47.2 |
| 8 | 194 | 52.1 |
| 13 | 163 | 48.2 |
| 15 | 175 | 47.9 |

Z01EM165 meets zeta potential stability test D requirements.

**Example 5**:

Methods:

| % (w/w) | Z01EM092 |
|---|---|
| CTAB | 0.005 |
| MCT | 1 |
| Vitamin E | 0.005 |

(continued)

| % (w/w) | Z01EM092 |
|---|---|
| Tyloxapol | 0.16 |
| Lutrol | 0.25 |
| Glycerin | 2.25 |
| Water | Up to 100 |

Preparation according to the process described in Reference Example 1.

Results:

| Emulsion | Z01EM092 | | | | | | |
|---|---|---|---|---|---|---|---|
| T(days) | 0 | 2 | 5 | 7 | 9 | 12 | 14 |
| Zeta potential (mV) | 19.8 | 21.9 | 22.4 | 18.5 | 20.3 | 18.5 | 20.5 |

Z01EM092 meets zeta potential stability test D requirements.

**Example 6**: **Cationic emulsions with BAK**

Methods:

| Emulsion | Z01EM105 | Z01EM155 | Z01EM162 | Z01EM163 |
|---|---|---|---|---|
| Components | %(w/w) | | | |
| BAK | 0.02 | 0.02 | 0.02 | 0.02 |
| MCT | 2 | 1 | 1 | 1 |
| Castor oil | 0 | 1 | 0 | 0 |
| Cremophor | 0 | 0.25 | 0.1 | 0 |
| Tyloxapol | 0.32 | 0 | 0.3 | 0.3 |
| Montane 20 | 0 | 0 | 0 | 0.1 |
| Lutrol | 0.5 | 0.1 | 0 | 0 |
| Oxypol | 0 | 0.25 | 0 | 0 |
| Vitamin E | 0.01 | 0 | 0 | 0 |
| Glycerin | 2.25 | 2.25 | 2.25 | 2.25 |
| water | Up to 100% | Up to 100% | Up to 100% | Up to 100% |

The stability of the autoclaved emulsions (droplet size, zeta potential) at 80°C was monitored T=0, 7 and 14 days.

Results:

|  | T(days) | Z01EM105 | Z01EM155 | Z01EM162 | Z01EM163 |
|---|---|---|---|---|---|
| Droplet size (nm) | 0 | nd | 288 | 243 | 249 |
|  | 7 | nd | 290 | 261 | 262 |
|  | 14 | nd | nd | 264 | 234 |
| Zeta potential (mV) | 0 | 24.9 | 19.7 | 22.3 | 18.8 |
|  | 2 | 20.7 | nd | nd | Nd |
|  | 7 | 21.3 | 14.2 | 14.7 | 16.5 |
|  | 10 | 23.2 | nd | nd | Nd |
|  | 13 | 22.2 | nd | nd | Nd |
|  | 14 | nd | nd | 17.4 | 15.7 |
|  | 15 | 23.2 | nd | nd | Nd |
| nd: not determined | | | | | |

Z01EM105, Z01EM162 and Z01EM163 meet zeta potential stability test D requirements. Z01EM115 meets.zeta potential stability test C requirements.

**Example 7**: **Cationic emulsions wherein the cationic agent is BEC or BCB**

Methods:

| Emulsion | Z01EM170 | Z01EM171 |
|---|---|---|
| components | %(w/w) |  |
| BEC | 0.02 | 0 |
| BCB | 0 | 0.02 |
| MCT | 2 | 2 |
| Tyloxapol | 0.3 | 0.3 |
| Glycerin | 2.25 | 2.25 |
| Lutrol | 0.1 | 0.1 |
| water | Up to 100% | Up to 100% |

Preparation according to the process described in Reference Example 1.

Results:

|  | T(days) | Z01EM170 | Z01EM171 |
|---|---|---|---|
| Droplet size (nm) | 0 | 210 | 239 |
|  | 7 | 232 | 250 |
|  | 14 | 233 | nd |

(continued)

| | T(days) | Z01EM170 | Z01EM171 |
|---|---|---|---|
| Zeta potential (mV) | 0 | 23.2 | 9.1 |
| | 7 | 22.4 | 6.4 |
| | 14 | 24.1 | 7.2 |

Z01EM170 and Z01EM171 meet zeta potential stability test D requirements.

**Example 8**: **Cationic emulsion with BAK and mineral oil** <u>Methods</u>:

| Emulsion | Z01EM151 | Z01EM152 | Z01EM153 | Z01EM164 | Z01EM173 |
|---|---|---|---|---|---|
| Components | % (w/w) | | | | |
| BAK | 0.02 | 0.02 | 0.02 | 0.02 | 0.02 |
| Mineral oil | 1 | 1 | 1 | 1 | 1 |
| Tyloxapol | 0.3 | 0.3 | 0.3 | 0.3 | 0.3 |
| Lutrol | 0.1 | 0.1 | 0.1 | 0.1 | 0.1 |
| Glycerin | 2.25 | 2.25 | 2.25 | 2.25 | 2.25 |
| Water | Up to 100% | Up to 100% | Up to 100% | 0 | 0 |
| Buffer | 0 | 0 | 0 | Up to 100% | Up to 100% |

The stability of the autoclaved emulsions (droplet size, zeta potential) at 80°C was monitored for 14 days.

<u>Results</u>:

| | T (days) | Z01EM151 | Z01EM152 | Z01EM153 | Z01EM164 | Z01EM173 |
|---|---|---|---|---|---|---|
| Droplet size (nm) | 0 | 25.8 | 26.0 | 23.4 | 16.7 | 20.7 |
| | 3 | 22.2 | 23.0 | 26.2 | 18.3 | nd |
| | 5 | 23.5 | 23.8 | 24.2 | nd | nd |
| | 7 | nd | nd | nd | nd | 19.7 |
| | 8 | nd | nd | nd | 16.5 | nd |
| | 10 | 34.1 | 20.4 | 17.7 | nd | nd |
| | 12 | 23.3 | 21.7 | 23.9 | nd | nd |
| | 13 | nd | nd | nd | 12.7 | nd |
| | 14 | 23.9 | 23 | 20.3 | nd | nd |
| | 15 | nd | nd | nd | 13.2 | 17.8 |

(continued)

| | T (days) | Z01EM151 | Z01EM152 | Z01EM153 | Z01EM164 | Z01EM173 |
|---|---|---|---|---|---|---|
| Zeta potential (mV) | 0 | 174 | 220 | 185 | 209 | 200 |
| | 3 | 178 | 215 | 184 | 200 | nd |
| | 5 | 170 | 213 | 182 | nd | nd |
| | 7 | nd | nd | nd | 213 | 200 |
| | 8 | nd | nd | nd | nd | nd |
| | 10 | 182 | 311 | 186 | nd | nd |
| | 12 | 176 | 221 | 224 | nd | nd |
| | 13 | nd | nd | nd | 218 | nd |
| | 14 | 168 | 216 | 188 | nd | nd |
| | 15 | nd | nd | nd | 203 | 216 |

All emulsions meet zeta potential stability test D requirements.

**Example 9**: **Cationic emulsions containing Cyclosporin, A (CsA) as active substance in the oil phase.**

Methods and results:

Preparation according to the process described in Reference Example 1, with the cyclosporin added to the oil phase.

The stability of the autoclaved emulsions (droplet size, zeta potential) at 80°C was monitored T=0, 7 and 15 days.

| Emulsion | Z06EM044 | Z06EM045 |
|---|---|---|
| Composition | 0.02% BAK<br>1.5% MCT<br>0.24% Tyloxapol<br>0.01% vit E<br>0.375% Lutrol<br>2.25% Glycerin<br>Water to 100%<br>CsA 0.1% | 0.02% BAK<br>2% MCT<br>0.32% Tyloxapol<br>0.01% vit E<br>0.5% Lutrol<br>2.25% Glycerin<br>Water to 100%<br>CsA 0.2% |
| Zeta potential (mV) | T0: 224<br>T7: 220<br>T15 239 | T0: 216<br>T7: 214<br>T15: 211 |
| Droplet size (nm) | T0: 25.3<br>T7: 22.9<br>T15: 21.8 | T0: 24.8<br>T7: 22.2<br>T15: 20.8 |

| Emulsion | Z06EM046 | Z06EM047 | Z06EM048 | Z06EM049 |
|---|---|---|---|---|
| Composition | 0.02% BAK<br>1% MCT<br>0.16% Tyloxapol<br>0.01% vit E<br>2.25% Glycerin<br>Water to 100%<br>CsA 0.05% | 0.02% BAK<br>0.75% MCT<br>0.12% Tyloxapol<br>0.01% vit E<br>0.1% Lutrol<br>2.25% Glycerin<br>Water to 100%<br>CsA 0.025% | 0.02% BAK<br>2% MCT<br>0.3% Tyloxapol<br>0.01% vit E<br>0.1% Lutrol<br>2.25% Glycerin<br>Water to 100%<br>CsA 0.025% | 0.02% BAK<br>1% MCT<br>0.16% Tyloxapol<br>0.01% vit E<br>0.1% Lutrol<br>2.25% Glycerin<br>Water to 100%<br>CsA 0.05% |
| Zeta potential (mV) | T0: 22.7<br>T7: 20.7<br>T15: 20.7 | T0: 20.9<br>T7: 18.9<br>T15: 16.9 | T0: 19.6<br>T7: 19.6<br>T15: 19.4 | T0: 25.0<br>T7: 24.7<br>T15: 19.2 |
| Droplet size (nm) | T0: 188<br>T7: 186<br>T15: 195 | T0: 165<br>T7: 188<br>T15: 194 | T0: 212<br>T7: 193<br>T15: 200 | T0: 159<br>T7: 173<br>T15: 177 |

| Emulsion | Z06EM050 | Z06EM051 | Z06EM052 | Z06EM053 |
|---|---|---|---|---|
| Composition | 0.02% BAK<br>2% MCT<br>0.3% Tyloxapol<br>0.01% vit E<br>0.1% Lutrol<br>2.25% Glycerin<br>Water to 100%<br>CsA 0.05% | 0.02% BAK<br>2% MCT<br>0.3% Tyloxapol<br>0.01% vit E<br>0.1% Lutrol<br>2.25% Glycerin<br>Water to 100%<br>CsA 0.2% | 0.02% BAK<br>4% MCT<br>0.3% Tyloxapol<br>0.01% vit E<br>0.1% Lutrol<br>2.25% Glycerin<br>Water to 100%<br>CsA 0.2% | 0.02% BAK<br>2% MCT<br>0.3% Tyloxapol<br>0.01% vit E<br>0.1% Lutrol<br>2.25% Glycerin<br>Water to 100%<br>CsA 0.1% |
| Zeta potential (mV) | T0: 17.9<br>T7: 20.3<br>T15: 18.9 | T0: 20.1<br>T7: 21.9<br>T15: 19.0 | T0: 28.4<br>T7: 24.5<br>T15: 23.0 | T0: 23.5<br>T7: 23.2<br>T15: 20.3 |
| Droplet size (nm) | T0: 224<br>T7: 212<br>T15: 221 | T0: 179<br>T7: 195<br>T15: 206 | T0: 176<br>T7: 201<br>T15: 195 | T0: 204<br>T7: 211<br>T15: 226 |

Emulsions containing CsA as active substance satisfy zeta potential stability test D requirements.

**Example 10**: **Cationic emulsion containing lutein as active ingredient.**

Method: As described previously.

|  | Z42EM001 | Z42EM002 |
|---|---|---|
| Lutein | 0.4 | 0.4 |
| Safflower oil | 1.6 | 1.6 |
| Tyloxapol | 0.3 | 0.3 |
| Montane 20 | - | 0.1 |
| BAK | 0.02 | 0.02 |
| Poloxamer | 0.1 | 0.1 |
| Glycerol | 2.25 | 2.25 |
| Deionised water | qsp 100 | qsp 100 |

Preparation according to the process described in Reference example 1.

|  |  | Z42EM001 | Z42EM002 |
|---|---|---|---|
| Droplet size (nm) | T0 | 347 | 255 |
|  | T7 | 290 | 236 |
|  | T14 | 285 | 321 |
| Zeta potential (mV) | T0 | 15.8 | 16.0 |
|  | T7 | 8.8 | 8.4 |
|  | T14 | 7.1 | 8.9 |

Emulsions containing lutein as active substance satisfy zeta potential stability test D requirements

**Example 11**: **Ocular tolerability test after chronic topical administration**

The aim of this study was to determine the ocular tolerance of cationic emulsions (Z01EM134, Z06EM048, Z06EM050 and Z06EM053; see composition in previous examples) after multiples daily ocular topical administrations for 28 consecutive days into the right eye of albino rabbits.

Methods:

Ten (10) New Zealand White albino rabbits per group (5 males and 5 females) were involved in this study. Treatments (50 $\mu$l ocular topical administrations) were given four times a day for 28 consecutive days. General tolerance (body weight, food and water consumptions, general aspect, clinical signs, hematology and blood biochemistry), ocular tolerance (observations with an ophthalmoscope, slit lamp examinations and ocular histology) and necropsy (gross macroscopic examination, main organ weights) were investigated. A statistical analysis (MANOVA LSD test) was also performed on body and organ weights, on food and water consumption data, and on hematological and biochemical parameters

Results:

General behaviour, food consumption and water consumption, body weight, organ weights were unaffected by treatments. There were no remarkable observations at necropsy due to treatment. Ophthalmological observations and microscopic examinations of the eyes and adnexa revealed no adverse effects. Ocular reactions were confined to slight conjunctival redness that were observed in all animals in the study and are commonly observed in rabbits after multiple instillations of ophthalmic products

**Claims**

1. An ophthalmic oil-in-water submicron type emulsion, which comprises colloid particles having an oily core surrounded by an interfacial film, said emulsion comprising:

   • 0.001 % to 0.1% by weight of at least one cationic agent selected from the group consisting of $C_{10}$-$C_{24}$ primary alkylamines, tertiary- aliphatic amines, quaternary ammonium compounds, cationic lipids, amino alcohols, biguanide salts, cationic polymers and a mixture of two or more thereof,
   • less than 1% by weight of at least one non ionic surfactant selected from the group consisting of tyloxapol, polysorbates, polyoxyethylene castor oil derivatives, sorbitan esters, polyoxyl stearates and a mixture of two or more thereof,
   • said emulsion having a positive zeta potential and metting the zeta potential stability Test A requirement as defined in the description,
   • provided that the emulsion does not contain phospholipids.

2. An ophthalmic emulsion according to claim 1, which meets the zeta potential stability Test B requirement as defined in the description.

3. An ophthalmic emulsion according to anyone of claim 1 or 2, which meets the zeta potential stability Test C requirement as defined in the description.

4. An ophthalmic emulsion according to anyone of claims 1 to 3, which meets the zeta potential stability Test D requirement as defined in the description.

5. An ophthalmic emulsion according to anyone of claims 1 to 4, wherein the concentration of the cationic agent is comprised between 0.002 and 0.05% w/w and still more preferably between 0.003 and 0.03% w/w.

6. An ophthalmic emulsion according to anyone of claims 1 to 5, wherein the concentration of the oil is not higher than 7% (w/w), preferably about 0.5 to 5% w/w, and even more preferably about 1% to 3%w/w.

7. An ophthalmic emulsion according to anyone of claims 1 to 6, wherein the weight ratio cationic agent/oil is comprised between 0.0025 and 0.06, preferably between 0.005 and 0.04.

8. An ophthalmic emulsion according to anyone of claims 1 to 7 wherein the concentration of the non-ionic surfactant is comprised preferably between 0.01 to 0.6% w/w.

9. An ophthalmic oil-in-water emulsion according to anyone of claims 1 to 8, wherein the cationic agent is selected in the group consisting of C10-C24 primary alkylamines, tertiary- aliphatic amines, quaternary ammonium compounds, cationic lipids, amino alcohols, biguanide salts, cationic polymers and a mixture of two or more thereof.

10. An ophthalmic oil-in-water emulsion according to claim 9, wherein the biguanide salt is selected from the group comprising chlorhexidine and salts thereof, polyaminopropyl biguanide, phenformin, alkylbiguanide or a mixture of two or more thereof.

11. An ophthalmic oil-in-water emulsion according to claim 9, wherein the quaternary ammonium compound is selected from the group comprising benzalkonium halide, lauralkonium halide, cetrimide, hexadecyltrimethylammonium halide, tetradecyltrimethylammonium halide, dodecyltrimethylammonium halide, cetrimonium halide, benzethonium halide, behenalkonium halide, cetalkonium halide, cetethyldimonium halide, cetylpyridinium halide, benzododecinium halide, chlorallyl methenamine halide, myristalkonium halide, stearalkonium halide or a mixture of two or more thereof, halide being preferably chloride or bromide.

12. An ophthalmic emulsion according to claim 11, wherein said cationic agent is selected from the group comprising benzalkonium chloride, lauralkonium chloride, benzododecinium bromide, benzethenium chloride, hexadecyltrimethylammonium bromide, tetradecyltrimethylammonium bromide, dodecyltrimethylammonium bromide or a mixture of two or more thereof.

13. An ophthalmic emulsion according to anyone of claims 1 to 9, wherein the cationic agent is selected from chitosan, 1,2- dioleyl-3- trimethylammonium-propane, 1, 2-dioleoyl-sn-glycero- phosphatidylethanolamine, cationic glycosphingo-lipids or cationic cholesterol derivatives, or mixtures of two or more thereof.

14. An ophthalmic emulsion according to anyone of claims 1 to 13 wherein the non-ionic surfactant is a mixture of two or more non-ionic surfactants selected from the group consisting of poloxamers, tyloxapol, polysorbates, polyoxyethylene castor oil derivatives, sorbitan esters, polyoxyl stearates.

15. An ophthalmic emulsion according to anyone of claims 1 to 14, comprising benzalkonium chloride as cationic agent and tyloxapol as non-ionic surfactant.

16. An ophthalmic emulsion according to anyone of claims 1 to 15, wherein the emulsion contains benzalkonium chloride as cationic agent and a combination of tyloxapol and poloxamer as non-ionic surfactants.

17. An ophthalmic emulsion according to anyone of claims 1 to 16, wherein said colloidal particles have an average particle size of equal or less than 1 $\mu$m, advantageously equal or less than 300 nm, more advantageously in the range of 100 to 250 nm.

18. An ophthalmic emulsion according to anyone of claims 1 to 17, comprising a pharmaceutically active substance.

19. An ophthalmic emulsion according to claim 18, wherein the active substance is selected from the group comprising antibiotics (such as tetracycline, chlortetracycline, bacitracin, neomycin, polymyxin, gramicidin, cephalexin, oxytetracycline, chloramphenicol, kanamycin, rifampicin, tobramycin, gentamycin, ciprofloxacin, aminosides, erythromycin and penicillin, quinolone, ceftazidime, vancomycine, imipeneme) ; antifungals such as amphotericin B and miconazole; antibacterials such as sulfonamides, sulfadiazine, sulfacetamide, sulfamethizole and sulfisoxazole, nitrofurazone and sodium propionate; antivirals, such as idoxuridine, trifluorotymidine, acyclovir, ganciclovir, cidofovir and interferon; non-antibiotic, anti-infection, antibacterial or anti-microbial drugs such as iodine based preparation triclosan, chlorhexidine; antiallergenics such as sodium cromoglycate, antazoline, methapyriline, chlorpheniramine, cetirizine, pyrilamine and prophenpyridamine; antiproliferative agents such as thalidomide; synthetic glucocorticoids and mineralocorticoids and more generally hormones forms derivating from the cholesterol metabolism (progesterone, estrogens, androgenic hormones such as testosterone, DHEA and their derivatives) / anti-inflammatories such as hydrocortisone, hydrocortisone acetate, dexamethasone, deicamethasone 21-phosphate, fluorocinolene, medrysone, prednisolone acetate, fluoromethalone, triamcinolone and triamcinolone acetonide and their derivatives; non-steroidal anti-inflammatories such as salicylate, indomethacin, ibuprofen, diclofenac, flurbiprofen and piroxicam and COX2 inhibitors such as rofecoxib, diclofenac, nimesulide, nepafenac; antineoplastics such as carmustine, cisplatin, mitomycin and fluorouracil; immunological drugs such as vaccines and immune stimulants; insulin, calcitonin, parathyroid hormone and peptide and vasopressin hypothalamus releasing factor; beta adrenergic blockers such as timolol maleate, levobunolol HCl and betaxolol HCl, timolol-base, betaxolol, atenolol, epinephrine, dipivalyl, oxonolol, acetazolamide-base and methazolamide; cytokines, interleukins, and growth factors (such as epidermal growth factor, fibroblast growth factor, platelet derived growth factor, transforming growth factor beta, ciliary neurotrophic growth factor, glial derived neurotrophic factor, NGF, EPO, P1GF) ; antibodies or antibodies fragments, oligoaptamers, aptamers and gene fragments (oligonucleotides, plasmids, ribozymes, small interference RNA, nucleic acid fragments, peptides, antisense sequences) ; immunosuppressants such as cyclosporine, sirolimus and tacrolimus, immunomodulators such as endoxan, tamoxifene; antithrombolytic and vasodilator agents such as rtPA, urokinase, plasmin, nitric oxide donors; antioxidants such as lutein, vitamins and/or their derivatives; and/or optically acceptable salts thereof.

20. An ophthalmic emulsion according to claim 18, wherein the active substance is an immunosuppressive agent selected in the group consisting of cyclosporine, sirolimus and tacrolimus.

21. An ophthalmic emulsion according to claim 20, wherein the active substance is cyclosporin A.

22. Process of preparation of a submicron ophthalmic oil-in-water type emulsion according to any one of claims 1 to 21, comprising the steps of shear mixing followed by high pressure homogenization of the coarse emulsions obtained through mixing of the aqueous and the oily phase.

23. Use of a submicron oil-in-water emulsion according to any one of claims 1 to 21, for the preparation of an ophthalmic composition for treating dry-eye conditions.

24. Use of an ophthalmic emulsion according to any one of claims 1 to 21, for the preparation of an ophthalmic composition for treating ocular conditions such as glaucoma, ocular inflammatory diseases such as keratitis, uveitis, intra-ocular inflammation, dry-eye syndrome, ocular infections, ocular allergies, cancerous growth, neo vessel growth originating from the cornea, retinal oedema, macular oedema, diabetic retinopathy, retinopathy of prematurity, degenerative diseases of the retina (macular degeneration, retinal dystrophies), retinal diseases associated with glial proliferation.

25. Ophthalmic formulation comprising a submicron emulsion according to any of claims 1 to 21, in combination with an ophthalmic acceptable carrier, said formulation being in the form of eye drops, eye ointment, or ophthalmic gel.

26. Ophthalmic formulation according to claim 25, comprising a pharmaceutically effective amount of an active ingredient in or within the ophthalmic acceptable carrier.

27. Delivery device selected from the group comprising lenses, ocular patch, implant, insert, said device containing an emulsion according to anyone of claims 1 to 21.

**Patentansprüche**

1. Ophthalmische Öl-in-Wasser-Submikron-Emulsion, die kolloidale Partikel mit einem durch einen Grenzflächenfilm umgebenen öligen Kern umfaßt, wobei die Emulsion folgendes umfaßt:

   • 0,001 bis 0,1 Gew.% von zumindest einem kationischen Mittel, das aus der Gruppe ausgewählt wird, die aus primären $C_{10}$-$C_{24}$-Alkylaminen, tertiären aliphatischen Aminen, quaternären Ammoniumverbindungen, kationischen Lipiden, Aminoalkoholen, Biguanidsalzen, kationischen Polymeren und einer Mischung von zwei oder mehreren davon besteht,
   • weniger als 1 Gew.% von zumindest einem nicht-ionischen Tensid, das aus der Gruppe ausgewählt wird, die aus Tyloxapol, Polysorbaten, Polyoxyethylen-Castoröl-Derivaten, Sorbitanestern, Polyoxylstearaten und einer Mischung aus zwei oder mehreren davon besteht,
   • wobei die Emulsion ein positives Zetapotential aufweist und das in der Beschreibung definierte Erfordernis des Zetapotential-Stabilitätstests A erfüllt,
   • unter der Maßgabe, daß die Emulsion keine Phospholipide enthält.

2. Ophthalmische Emulsion gemäß Anspruch 1, die das in der Beschreibung definierte Erfordernis des Zetapotential-Stabilitätstests B erfüllt.

3. Ophthalmische Emulsion gemäß mindestens einem der Ansprüche 1 oder 2, die das in der Beschreibung definierte Erfordernis des Zetapotential-Stabilitätstests C erfüllt.

4. Ophthalmische Emulsion gemäß mindestens einem der Ansprüche 1 bis 3, die das in der Beschreibung definierte Erfordernis des Zetapotential-Stabilitätstests D erfüllt.

5. Ophthalmische Emulsion gemäß mindestens einem der Ansprüche 1 bis 4, worin die Konzentration des kationischen Mittels zwischen 0,002 und 0,05 % G/G und besonders bevorzugt zwischen 0,003 und 0,03 % G/G liegt.

6. Ophthalmische Emulsion gemäß mindestens einem der Ansprüche 1 bis 5, worin die Konzentration des Öls nicht höher als 7 % (G/G), vorzugsweise etwa 0,5 bis 5 % G/G und besonders bevorzugt etwa 1 bis 3 % G/G ist.

7. Ophthalmische Emulsion gemäß mindestens einem der Ansprüche 1 bis 6, worin das Gewichtsverhältnis kationisches Mittel/Öl zwischen 0,0025 und 0,06, vorzugsweise zwischen 0,005 und 0,04 liegt.

8. Ophthalmische Emulsion gemäß mindestens einem der Ansprüche 1 bis 7, worin die Konzentration des nicht-ionischen Tensid vorzugsweise zwischen 0,01 und 0,6 % G/G liegt.

9. Ophthalmische Öl-in-Wasser-Emulsion gemäß mindestens einem der Ansprüche 1 bis 8, worin das kationische Mittel aus der Gruppe ausgewählt wird, die aus primären $C_{10}$-$C_{24}$-Alkylaminen, tertiären aliphatischen Aminen, quaternären Ammoniumverbindungen, kationischen Lipiden, Aminoalkoholen, Biguanidsalzen, kationischen Polymeren und einer Mischung von zwei oder mehreren davon besteht.

**10.** Ophthalmische Öl-in-Wasser-Emulsion gemäß Anspruch 9, worin das Biguanidsalz aus der Gruppe ausgewählt wird, die Chlorhexidin und Salze davon, Polyaminopropylbiguanid, Phenformin, Alkylbiguanid oder eine Mischung von zwei oder mehreren davon umfaßt.

**11.** Ophthalmische Öl-in-Wasser-Emulsion gemäß Anspruch 9, worin die quaternäre Ammoniumverbindung aus der Gruppe ausgewählt wird, die Benzalkoniumhalogenid, Lauralkoniumhalogenid, Cetrimid, Hexadecyltrimethylammoniumhalogenid, Tetradecyltrimethylammoniumhalogenid, Dodecyltrimethylammoniumhalogenid, Cetrimoniumhalogenid, Benzethoniumhalogenid, Behenalkoniumhalogenid, Cetalkoniumhalogenid, Cetetyldimoniumhalogenid, Cetylpyridiniumhalogenid, Benzododeciniumhalogenid, Chlorallylmethenaminhalogenid, Myristalkoniumhalogenid, Stearalkoniumhalogenid oder eine Mischung von zwei oder mehreren davon umfaßt, wobei das Halogenid vorzugsweise Chlorid oder Bromid ist.

**12.** Opthalmische Emulsion gemäß Anspruch 11, worin das kationische Mittel aus der Gruppe ausgewählt wird, die Benzalkoniumchlorid, Lauralkoniumchlorid, Benzododeceniumbromid, Benzetheniumchlorid, Hexadecyltrimethylammoniumbromid, Tetradecyltrimethylammoniumbromid, Dodecyltrimethylammoniumbromid oder eine Mischung von zwei oder mehreren davon umfaßt.

**13.** Ophthalmische Emulsion gemäß mindestens einem der Ansprüche 1 bis 9, worin das kationische Mittel aus Chitosan, 1,2-Dioleyl-3-trimethylammonium-propan, 1,2-Dioleoyl-sn-glycero-phosphatidylethanolamin, kationischen Glycosphingolipiden oder kationischen Cholesterinderivaten oder einer Mischung von zwei oder mehreren davon ausgewählt wird.

**14.** Ophthalmische Emulsion gemäß mindestens einem der Ansprüche 1 bis 13, worin das nicht-ionische Tensid eine Mischung von zwei oder mehreren nicht-ionischen Tensiden ist, die aus der Gruppe ausgewählt werden, die aus Poloxameren, Tyloxapol, Polysorbaten, Polyethylen-Castoröl-Derivaten, Sorbitanestern, Polyoxylstearaten besteht.

**15.** Ophthalmische Emulsion gemäß mindestens einem der Ansprüche 1 bis 14, umfassend Benzalkoniumchlorid als kationisches Mittel und Tyloxapol als nicht-ionisches Tensid.

**16.** Ophthalmische Emulsion gemäß mindestens einem der Ansprüche 1 bis 15, wobei die Emulsion Benzalkoniumchlorid als kationisches Mittel und eine Kombination aus Tyloxapol und Poloxamer als nicht-ionische Tenside enthält.

**17.** Ophthalmische Emulsion gemäß mindestens einem der Ansprüche 1 bis 16, worin die kolloidalen Partikel eine durchschnittlichen Partikelgröße von gleich oder weniger als 1 $\mu$m, vorteilhafterweise gleich oder weniger als 300 nm, besonders vorteilhaft im Bereich von 100 bis 250 nm, aufweisen.

**18.** Ophthalmische Emulsion gemäß mindestens einem der Ansprüche 1 bis 17, umfassend eine pharmazeutisch wirksame Substanz.

**19.** Ophthalmische Emulsion gemäß Anspruch 18, worin die wirksame Substanz aus der Gruppe ausgewählt wird, die Antibiotika (wie Tetracyclin, Chlortetracyclin, Bacitracin, Neomycin, Polymyxin, Gramicidin, Cephalexin, Oxytetracyclin, Chloremphenicol, Kanamycin, Rifampicin, Tobramycin, Gentamycin, Ciprofloxacin, Aminoside, Erythromycin und Penicillin, Chinolon, Ceftazidim, Vancomycin, Imipenem); Antimykotika wie Amphotericin B und Miconazol; antibakterielle Substanzen wie Sulfonamide, Sulfadiazin, Sulfacetamid, Sulfamethizol und Sulfisoxazol, Nitrofurazon und Natriumpropionat; Viruzide wie Idoxuridin, Trifluortymidin, Acyclovir, Ganciclovir, Cidofovir und Interferon; Nicht-Anbitiotika, Anti-Infektionsmittel, antibakterielle oder antimikrobielle Arzneimittel, wie eine Zubereitung auf Jodbasis, Triclosan, Chlorhexidin; Antiallergika wie Natriumcromoglycat, Antazolin, Methapyrilin, Chlorpheniramin, Cetirizin, Pyrilamin und Prophenpyridamin; antiproliferative Mittel wie Thalidomid; synthetische Glucocorticoide und Mineralocorticoide und allgemeine Hormonformen, die aus dem Cholesterinmetabolismus abgeleitet werden (Progesteron, Östrogene, Androgene wie Testosteron, DHEA und ihre Derivate)/entzündungshemmende Mittel wie Hydrocortison, Hydrocortisonacetat, Dexamethason, Dexamethason-21-phosphat, Fluorcinolen, Medryson, Prednisolonacetat, Fluormethalon, Triamcinolon und Triamcinolenacetonid und ihre Derivate; nichtsteroidale entzündungshemmende Mittel wie Salicylat, Indomethacin, Ibuprofen, Diclofenac, Flurbiprofen und Piroxicam und COX2-Inhibitoren wie Rofecoxib, Diclofenac, Nimesulid, Nepafenac; antineoplastische Mittel wie Carmustin, Cisplatin, Mitomycin und Fluroruracil; immunologische Arzneimittel wie z.B. Vakzine und Immunstimulanzien; Insulin, Calcitonin, Parathormon und -peptid und Vasopressin-Hypothalamus-Releasingfaktor; beta-Adrenorezeptor-Antagonisten wie Timololmaleat, Levobunolol-HCl und Hetaxolo-HCl, Timolol-Base, Betaxolol, Atenolol, Epinephrin, Dipivalyl, Oxonolol, Acetazolamid-Base und Methazolamid; Zytokine, Interleukine und Wachstumsfaktoren (wie der epidermale Wachstumsfaktor, Fibroblasten-Wachstumsfaktor, Blutplättchenabgeleiteter Wachstumsfaktor, transformierender Wachstumsfaktor beta, ciliary neurotrophic growth factor, glial derived neurotrophic factor, NGF, EPO, PIGF); Antikörper und Antikörperfragmente, Oligoaptamere, Aptamere und Genfragmente (Oligonukleotide, Plasmide, Ribozyme, small interference RNA, Nukleinsäurefragmente, Peptide, Antisense-Sequenzen); Immunosuppressiva wie Cyclosporin, Sirolimus und Tacrolimus, Immunomodulatoren wie Endoxan, Tamoxifen; Antithrombotika und Vasodilatoren wie rtPA, Urokinase, Plasmin, Stickoxiddonatoren; Antioxidantien wie Lutein, Vitamine und/oder ihre Derivate; und/oder optisch annehmbare Salze davon umfaßt.

20. Ophthalmische Emulsion gemäß Anspruch 18, worin die wirksame Substanz ein Immunosuppressivum ist, das aus der Gruppe ausgewählt wird, die aus Cyclosporin, Sirolimus und Tacrolimus besteht.

21. Ophthalmische Emulsion gemäß Anspruch 20, worin die wirksame Substanz Cyclosporin A ist.

22. Verfahren zur Herstellung einer ophthalmischen Öl-in-Wasser-Submikron-Emulsion gemäß mindestens einem der Ansprüche 1 bis 21, umfassend die Schritte des Schermischens gefolgt durch Hochdruck-Homogenisierung der durch Mischen der wäßrigen und der öligen Phase erhaltenen groben Emulsionen.

23. Verwendung einer Öl-in-Wasser-Submikron-Emulsion gemäß mindestens einem der Ansprüche 1 bis 21 zur Herstellung einer ophthalmischen Zusammensetzung zur Behandlung von Benetzungsstörungen.

24. Verwendung einer ophthalmischen Emulsion gemäß mindestens einem der Ansprüche 1 bis 21 zur Herstellung einer ophthalmischen Zusammensetzung zur Behandlung von Augenerkrankungen, wie Glaukom, Augenentzündungserkrankungen wie Hornhautentzündung, Uveitis, Innenaugenentzündung, trockenes-Auge-Syndrom, Augeninfektionen, Augenallergien, kanzerösem Wachstum, aus der Hornhaut stammendem Neo-Gefäßwachstum, Netzhautödem, Makulaödem, diabetischer Retinopathie, Retinopathia praematurorum, degenerativen Erkrankungen der Netzhaut (Makuladegeneration, Netzhautdystrophien), mit Glialproliferation verbundene Netzhauterkrankungen.

25. Ophthalmische Formulierung, umfassend eine Submikron-Emulsion gemäß mindestens einem der Ansprüche 1 bis 21 in Kombination mit einem ophthalmisch annehmbaren Träger, wobei die Formulierung in Form von Augentropfen, einer Augensalbe oder einem opthalmischen Gel vorliegt.

26. Ophthalmische Formulierung gemäß Anspruch 25, umfassend eine pharmazeutisch wirksame Menge eines Wirkstoffs in oder innerhalb des ophthalmisch annehmbaren Trägers.

27. Applikationsvorrichtung, die aus der Gruppe ausgewählt wird, die Linsen, ein Augenpflaster, ein Implantat, einen Einsatz umfaßt, wobei die Vorrichtung eine Emulsion gemäß mindestens einem der Ansprüche 1 bis 21 enthält.

**Revendications**

1. Emulsion ophtalmique huile-dans-eau de type submicronique, qui comprend des particules colloïdales ayant un coeur huileux entouré d'un film interfacial, ladite émulsion comprenant :

   • De 0,001 % à 0,1 % en poids d'au moins un agent cationique sélectionné dans le groupe constitué des alkylamines primaires en $C_{10}$-$C_{24}$, des amines aliphatiques tertiaires, des composés ammonium quaternaires, des lipides cationiques, des amino alcools, des sels de biguanide, des polymères cationiques et un mélange de deux ou plus de ceux-ci,
   • Moins de 1 % en poids d'au moins un surfactant non-ionique sélectionné dans le groupe constitué du tyloxapol, des polysorbates, des dérivés de l'huile de ricin polyoxyéthylénée, des esters de sorbitan, des stéarates polyoxyéthyléniques et un mélange de deux ou plus de ceux-ci,
   • Ladite émulsion ayant un potentiel zêta positif et satisfaisant aux exigences du test A de stabilité du potentiel zêta tel que défini dans la description,
   • A condition que l'émulsion de contienne pas de phospholipides.

2. Emulsion ophtalmique selon la revendication 1, qui satisfait aux exigences du test B de stabilité du potentiel zêta tel que défini dans la description.

3. Emulsion ophtalmique selon la revendication 1 ou 2, qui satisfait aux exigences du test C de stabilité du potentiel zêta tel que défini dans la description.

4. Emulsion ophtalmique selon l'une quelconque des revendications 1 à 3, qui satisfait aux exigences du test D de stabilité du potentiel zêta tel que défini dans la description.

5. Emulsion ophtalmique selon l'une quelconque des revendications 1 à 4, dans laquelle la concentration de l'agent cationique est comprise entre 0,002 et 0,05 % m/m et plus préférentiellement entre 0,003 et 0,03 % m/m.

6. Emulsion ophtalmique selon l'une quelconque des revendications 1 à 5, dans laquelle la concentration de l'huile n'est pas supérieure à 7 % (m/m), de préférence environ 0,5 à 5 % m/m, et plus préférentiellement entre 1 et 3 % m/m.

7. Emulsion ophtalmique selon l'une quelconque des revendications 1 à 6, dans laquelle le ratio agent cationique / huile est compris entre 0,0025 et 0,06, de préférence entre 0,005 et 0,04.

8. Emulsion ophtalmique selon l'une quelconque des revendications 1 à 7, dans laquelle la concentration de l'agent non-ionique est comprise de préférence entre 0,01 et 0,6 % m/m.

9. Emulsion ophtalmique selon l'une quelconque des revendications 1 à 8, dans laquelle l'agent cationique est sélectionné dans le groupe constitué des alkylamines primaires en $C_{10}$-$C_{24}$, des amines aliphatiques tertiaires, des composés ammonium quaternaires, des lipides cationiques, des amino alcools, des sels de biguanide, des polymères cationiques et un mélange de deux ou plus de ceux-ci.

10. Emulsion ophtalmique selon la revendication 9, dans laquelle le sel de biguanide est sélectionné dans le groupe comprenant la chlorhexidine et ses sels, le polyaminopropyl biguanide, la phenformine, l'alkylbiguanide ou un mélange de deux ou plus de ceux-ci.

11. Emulsion ophtalmique selon la revendication 9, dans laquelle le composé ammonium quaternaire est sélectionné dans le groupe comprenant de l'halogénure de benzalkonium, de l'halogénure de lauralkonium, du cetrimide, de l'halogénure d'hexadécyltriméthylammonium, de l'halogénure de tétradécyltriméthylammonium, de l'halogénure de dodécyltriméthylammonium, de l'halogénure de cétrimonium, de l'halogénure de benzéthonium, de l'halogénure de béhénalkonium, de l'halogénure de cétalkonium, de l'halogénure de cététhyldimonium, de l'halogénure de cétylpyridinium, de l'halogénure de benzododécinium, de l'halogénure de chlorallyl méthénamine, de l'halogénure de myristalkonium, de l'halogénure de stéaralkonium ou un mélange de deux ou plus de ceux-ci, l'halogénure étant de préférence du chlorure ou du bromure.

12. Emulsion ophtalmique selon la revendication 11, dans laquelle ledit agent cationique est sélectionné dans le groupe comprenant le chlorure de benzalkonium, le chlorure de lauralkonium, le bromure de benzododecinium, le chlorure de benzéthénium, le bromure d'héxadécyltriméthylammonium, le bromure de tétradécyltriméthylammonium, le bromure de dodécyltriméthylammonium ou un mélange de deux ou plus de ceux-ci.

13. Emulsion ophtalmique selon l'une quelconque des revendications 1 à 9, dans laquelle l'agent cationique est sélectionné parmi le chitosane, le 1,2- dioleyl-3-triméthylammonium-propane, le 1, 2-dioleoyl-sn-glycéro- phosphatidyléthanolamine, les glycosphingo-lipides cationiques ou les dérivés du cholestérol cationiques un mélange de deux ou plus de ceux-ci.

14. Emulsion ophtalmique selon l'une quelconque des revendications 1 à 13, dans laquelle le surfactant non-ionique est un mélange de deux ou plus surfactants non-ioniques sélectionnés dans le groupe constitué des poloxamères, du tyloxapol, des polysorbates, des dérivés de l'huile de ricin polyoxyéthylénée, des esters de sorbitan, des stéarates polyoxyéthyléniques et un mélange de deux ou plus de ceux-ci.

15. Emulsion ophtalmique selon l'une quelconque des revendications 1 à 14, comprenant du chlorure de benzalkonium comme agent cationique et du tyloxapol comme surfactant non-ionique.

16. Emulsion ophtalmique selon l'une quelconque des revendications 1 à 15, dans laquelle l'émulsion contient du chlorure de benzalkonium comme agent cationique et une combinaison de tyloxapol et de poloxamère comme surfactants non-ioniques.

17. Emulsion ophtalmique selon l'une quelconque des revendications 1 à 16, dans laquelle lesdites particules colloïdales ont une taille de particule moyenne inférieure ou égale à 1 µm, avantageusement inférieure ou égale à 300 nm, plus avantageusement dans la gamme allant de 100 à 250 nm.

18. Emulsion ophtalmique selon l'une quelconque des revendications 1 à 17, comprenant une substance pharmaceutiquement active.

19. Emulsion ophtalmique selon la revendication 18, dans laquelle la substance active est sélectionnée dans le groupe comprenant les antibiotiques (tels que tétracycline, chlortétracycline, bacitracine, néomycine, polymyxine, gramicidine, céphalexine, oxytétracycline, chloramphénicol, kanamycine, rifampicine, tobramycine, gentamycine, ciprofloxacine, aminosides, érythromycine et pénicilline, quinolone, ceftazidime, vancomycine, imipénème); antifongiques tels que amphotéricine B et miconazole; antibactériens tels que sulfonamides, sulfadiazine, sulfacétamide, sulfaméthizole et sulfisoxazole, nitrofurazone et propionate de sodium; antiviraux, tels qu'idoxuridine, trifluorotymidine, aciclovir, ganciclovir, cidofovir et interféron; médicaments non-antibiotiques, anti-infectieux, antibactériens ou anti-microbiens tels que les préparations à base diode, triclosan, chlorhexidine; antiallergéniques tels que cromoglycate de sodium, antazoline, méthapyriline, chlorphéniramine, cétirizine, pyrilamine et prophenpyridamine; agents antiprolifératifs tels que thalidomide; glucocorticoïdes synthétiques et minéralocorticoïdes et plus généralement les formes hormonales dérivant du métabolisme du cholestérol (progestérone, estrogènes, hormones androgéniques telles que la testostérone, DHA et leurs dérivés) / anti-inflammatoires tels que hydrocortisone, acétate d'hydrocortisone, dexaméthasone, dexaméthasone 21-phosphate, fluorocinolène, médrysone, acétate de prednisolone, luorométhalone, triamcinolone et acétonide de triamcinolone et leurs dérivés; anti-inflammatoires non-stéroïdiens tels que salicylate, indométhacine, ibuprofène, diclofénac, flurbiprofène et piroxicam et inhibiteurs COX2 tels que rofécoxib, diclofénac, nimésulide, népafénac; anticancéreux tels que carmustine, cisplatine, mitomycine et fluorouracile; médicaments immunologiques tels que vaccins et stimulants immunitaires; insuline, calcitonine, hormone parathyroïde et peptide et facteur de libération de la vasopressine hypothalamique; beta-bloquants tels que maléate de timolol, hydrochlorure de lévobunolol et hydrochlorure de bétaxolol, timolol-base, bétaxolol, aténolol, épinéphrine, dipivalyl, oxonolol, acétazolamide-base et méthazolamide; cytokines, interleukines, and facteurs de croissance (tels que facteur de croissance de l'épiderme, facteur de croissance du fibroblastes, facteur de croissance dérivé des plaquettes, facteur de croissance transformant β, facteur de croissance neurotrophique ciliaire, facteur neurotrophique dérivé des cellules gliales, NGF, EPO, P1GF); anticorps ou des fragments d'anticorps, oligoaptamères, aptamères et des fragments de gène (oligonucléotides, plasmides, ribozymes, ARN interférant, acide nucléique, peptides, séquences antisense); immunosuppresseurs tels que cyclosporine, sirolimus et tacrolimus, immunomodulateurs tels qu'endoxane, tamoxifène; antithrombolytique et agents vasodilatateurs tels que rtPA, urokinase, plasmine, donneurs d'oxyde nitrique; antioxydants tels que lutéine, vitamines et/ou leurs dérivés; et/ou les sels optiquement acceptable de ceux-ci.

20. Emulsion ophtalmique selon la revendication 18, dans laquelle la substance active est un agent immunosuppresseur sélectionné dans le groupe constitué de la cyclosporine, du sirolimus et du tacrolimus.

21. Emulsion ophtalmique selon la revendication 20, dans laquelle la substance active est la cyclosporine A.

22. Procédé de préparation d'une émulsion ophtalmique huile-dans-eau de type submicronique selon l'une quelconque des revendications 1 à 21, comprenant les étapes de cisaillement suivi d'une homogénéisation à haute pression des émulsions grossières obtenues par le mélange de la phase aqueuse et de la phase huileuse.

23. Utilisation de l'émulsion ophtalmique huile-dans-eau de type submicronique selon l'une quelconque des revendications 1 à 21, pour la préparation d'une composition ophtalmique pour le traitement des maladies de l'oeil sec.

24. Utilisation de l'émulsion ophtalmique huile-dans-eau de type submicronique selon l'une quelconque des revendications 1 à 21, pour la préparation d'une composition ophtalmique pour le traitement de maladies oculaires telles que le glaucome, les maladies inflammatoires oculaires telles la kératite, l'uvéite, l'inflammation intra-oculaire, le syndrome de l'oeil sec, les infections oculaires, les allergies oculaires, la croissance cancéreuse, la croissance de néovaisseaux provenant de la cornée, oedème de la rétine, oedème maculaire, la rétinopathie diabétique, la rétinopathie des prématurés, les maladies dégénératives de la rétine (dégénération maculaire, dystrophie rétinienne), les maladies rétiniennes associés à la prolifération gliale.

25. Formulation ophtalmique comprenant une émulsion submicronique selon l'une quelconque des revendications 1 à 21, en combinaison avec un véhicule ophtalmique acceptable, ladite formulation étant sous la forme de gouttes ophtalmiques, de pommade ophtalmique ou de gel ophtalmique.

**26.** Formulation ophtalmique selon la revendication 25, comprenant une quantité pharmaceutiquement efficace d'un ingrédient actif dans ou à l'intérieur du véhicule ophtalmique acceptable.

**27.** Dispositif d'administration sélectionné dans le groupe comprenant les lentilles, le patch oculaire, l'implant, l'insert, ledit dispositif contenant une émulsion selon l'une quelconque des revendications 1 à 21.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6007826 A
- US 6656460 B, Benita and Lambert
- WO 03053405 A
- EP 0696452 A
- EP 0878197 A
- EP 1611879 A

**Non-patent literature cited in the description**

- **KLANG et al.** *Pharm. Dev. Technology,* 2000, vol. 5, 521-532
- **WASHINGTON.** *Adv. Drug Deliv. Reviews,* 1996, vol. 20, 131-145
- **RABINOVICH-GUILATT et al.** *Chem Phys Lipids,* 2004, vol. 131, 1-13
- **LIU et al.** *Pharm. Res.,* 1996, vol. 13, 1856-1860
- **KLANG et al.** *Int. J. Pharm.,* 1996, vol. 132, 33-44
- **ZUIDAM ; CROMMELIN.** *J Pharm Sci,* 1995, vol. 84, 1113-1119
- **VARVERI et al.** *J. Photochem. Photobiol. A,* 1995, vol. 91, 121-124
- **TAMILVANAN et al.** *STP Pharma Sciences,* 2001, vol. 11, 421-426
- *Int. J Pharm.,* 1999, vol. 183, 175-84
- *Eur. J. Pharm. Biopharm.,* 2002, vol. 53, 115-23
- **KLANG et al.** *J. Pharm. Pharmacol.,* 1994, vol. 46, 986-993
- **FURRER et al.** *Eur. J. Pharm. Biopharm.,* 2002, vol. 53, 263-280